# EUROPEAN PATENT SPECIFICATION

(11) **EP 0 871 758 B1**
(45) Date of publication and mention of the grant of the patent: **31.03.2004**
(21) Application number: 96938254.8
(22) Date of filing: 20.11.1996
(51) Int. Cl.: C12N 15/88

(54) **LIPOSOMAL TRANSFECTION METHOD**
TRANSFEKTIONSVERFAHREN MIT HILFE VON LIPOSOMEN
PROCEDE DE TRANSFECTION PAR LIPOSOMES

(30) Priority: 21.11.1995 US 560199
(43) Date of publication of application: 21.10.1998
(73) Proprietor: KINNUNEN, Paavo Kai Johannes, SF-02660 Espoo (FI); Paukku, Tommi, 20540 Turku (FI); Lauraeus, Satu, 00530 Helsinki (FI); Huhtaniemi, Ilpo, 20540 Turku (FI)
(72) Inventor: KINNUNEN, Paavo Kai Johannes, SF-02660 Espoo (FI); Paukku, Tommi, 20540 Turku (FI); Lauraeus, Satu, 00530 Helsinki (FI); Huhtaniemi, Ilpo, 20540 Turku (FI)
(74) Representative: Grew, Eva Regina
(86) International application number: PCT/FI1996/000629
(87) International publication number: WO 1997/019184

(56) References cited:
- WO-A-87/07530
- WO-A-95/26356
- US-A- 5 279 833

## Description

### FIELD OF INVENTION

The present invention relates to a method of transfecting a cell with nucleic acid, such as DNA, using, as the transfection vector, a liposomal composition comprising a sphingosine derivative having a protonated amino group in the sphingosine moiety. The protonated amino group provides for the required cationic nature of the liposome for forming a complex with the negatively charged nucleic acid. According to the invention it was shown that the sphingosine containing liposomal composition, which had been pre-incubated with nucleic acid, such as DNA, transfected efficiently the cell lines tested.

### BACKGROUND OF THE INVENTION

Liposomes are widely used for drug delivery and for delivery of foreign DNA to mammalian cells, and they offer several advantages in this respect. Such advantages are ease of manufacture, commercial availability, the possibility of targeting to specific cells or tissues, and high transfection efficiency. Cationic liposomes, that is liposomes comprising at least some positively charged lipids to give an overall positive charge to the liposomes, have been used for gene transfer, i.e. transfection of DNA, into mammalian cells both *in vivo* and *in vitro.* Due to charge interactions, the positively charged liposome forms easily, in a simple mixing process, on its surface a complex with the negatively charged DNA. The complex in turn binds strongly to the cell surface due to favorable charge interactions followed by internalization of the complex into the cell and expression of the gene (Singhal, A. and Huang, L. (1994) *Gene Therapeutics* (ed.: Wolff, J.A.) pp 118-142, Birkhauser, Boston).

Various cationic lipids have been suggested in the art for incorporation into liposomes, including quaternary ammonium detergents, e.g. cetyltrimethylammonium bromide (CTAB), cationic derivatives of cholesterol and diacyl glycerol, e.g. 1,2-dioleyl-3-(4'-trimethylammonio)butanoyl-sn-glycerol (DOTB), and lipopolyamines, e.g. lipopoly-L-lysine (LPLL). Also commercial preparations are available. Lipofectin^{•} is a widely used commercial preparation comprising N-[1-(2,3-dioleyloxy)propyl]-N,N,N-trimethylammonium chloride (DOTMA) in combination with dioleoylphosphatidylethanolamine (DOPE) (Gibco). The apparent toxicity of some of the commercially available products have been referred to the non-natural, non-biodegradable nature of compounds contained by them (Singhal et al., *ibid*).

The cationic liposomes used as a transfection vector commonly contain, in addition to the cationic lipid, a neutral or negatively charged lipid, so called helper lipid or co-lipid, the above mentioned DOPE being one such neutral helper lipid. Also other helper lipids have been suggested. Such helper lipids are typically phospholipids, and besides DOPE, e.g. dioleoylphosphatidylcholine (DOPC), dioleoylphatidylserine (DOPS), the corresponding dilauroyl, dimyristoyl and dipalmitoyl compounds, phosphatidic acid, phosphatidylglycerol, sterols, such as cholesterol, and mixtures of these have been suggested. The mono-, di- and triglycerides may be mentioned as further neutral helper lipids. A main function of the helper lipid is to fuse into and stabilize the liposome bilayer structure. It is also known that DOPE, in addition to the liposomal stabilizing effect, aids in the cytoplasmic delivery of DNA in the cell.

A wide variety of patent literature is available disclosing various cationic lipids for use for transfection, see e.g. US 5,264,618, WO 93/03709, and WO 95/17373, just to mention a few. WO 87/07530 discloses liposomes containing i.a. sphingosine WO 85/26356 discloses the use of phospholipids for transfection.

Although a number of advantages are obtained by using cationic liposomes as carriers for DNA, rather than neutral or anionic liposomes, many problems still remain both in *in vivo* and *in vitro* applications. A major problem with many of the cationic lipids is that they are generally toxic to the cells, and thus of limited use. This is especially true of Lipofectin^{•}, the DOTMA component of which is a diether, and not readily degraded *in vivo* and toxic to tissue cells. Thus DOTMA is not optimal from the point of view of *in vivo* gene delivery.

According to the invention it has now been discovered that by using a specific group of cationic lipids for inclusion into the transfection vector, it is possible to provide a method of transfection displaying clearly improved transfection efficiencies with minimal toxicity problems to the transfected cells.

### SUMMARY OF THE INVENTION

The object of the present invention is thus a method for transfecting a cell with a nucleic acid which comprises contacting the cell with a transfection composition comprising the nucleic acid and liposomes, the nucleic acid being complexed to the surface of said liposomes, the liposome comprising
- sphingosine or a derivative thereof, having a protonated amino group in the sphingosine moiety, and
- a helper lipid.

### DETAILED DESCRIPTION OF THE INVENTION

Sphingosine derivatives are amphiphilic lipids, i.e. they comprise an amino alcohol substituted with a single fatty chain. Several sphingosine derivatives occur naturally in mammalian and non-mammalian cells, and they can be degraded in connection with normal cellular metabolism. The role of sphingosines in influencing DNA synthesis and gene expression is, however, largely unknown. It is also known that sphingosine and some of its cationic derivatives in liposomal form bind strongly to DNA (Koiv, A. and Kinnunen, P.K.J., *Chem. Phys. Lipids,* 72 (1994) 77-86).

Another feature of the sphingosine is its ability to inhibit the protein kinase C (PKC). The inhibition of PKC has been proposed to be a critical factor determining the transfection efficiency and toxicity of a given liposome vector (Singhal et al., *ibid*. p. 125). Sphingosine containing liposomes have not been suggested for use in transfection vectors, apparently due to the fact that sphingosine, being a PKC inhibitor, would be expected to resist transfection. According to the invention it has now, however, been discovered that this is not the case, and that substantially enhanced transfection efficiencies, without appended toxicity to the cells, can be obtained, as compared to using Lipofectin^{•}, by including, in the liposome vector, at least one positively charged sphingosine derivative.

The main criteria when choosing the sphingosine derivative to be used according to the invention is that the amino group in the sphingosine moiety is in protonable form, in order to provide for the necessary overall cationic nature to the liposome vector formed by the sphingosine compound and the helper lipid. The cationic nature of the liposome in turn is critical for the efficient binding of the negatively charged nucleic acid to the surface of the liposome. Especially contemplated for use in the invention are sphingosine and the sphingosine derivatives having the following general formula 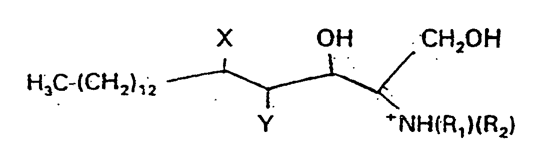 wherein
X and Y are each, independently of each other, H or OH, or together form a double bond,
R₁ and R₂ are independently H or a lower alkyl group with 1-3 carbon atoms.

A lower alkyl group as R₁ and/or R₂ is one with 1-3 carbon atoms, preferably methyl.

X is preferably H or forms a double bond with Y.

Especially contemplated for use in the invention are sphingosine, phytosphingosine, dihydrosphingosine and dimethylsphingosine.

According to the invention, the liposomal transfection vector incleldes also at least one helper lipid, which is not a sphingosine derivative. Such a lipid is typically selected. from the group of neutral phospholipids, typically it is phosphatidylethanolamine, or a diacylglycerol. The helper lipid has fusogenic properties meaning that the lipid has the property of facilitating the fusion of lipid membranes, see e.g. Kinnunen, P. K. J., *Chem. Phys. Lipids.,* 63 (1992)251-258. However, within the context of the invention, any lipid which has fusogenic properties and which is neutral or which does not substantially alter the cationic nature of the formed liposome, can come into question.

According to a preferred embodiment of the invention the composition comprises a first helper lipid which is selected from fusogenic neutral phospholipids, especially from phosphatidylethanolamines, and a second helper lipid selected from diacyl glycerols.

A preferred phospholipid is phosphatidylethanolamine, especially dioleoylphosphatidylethanolamine (DOPE), or the corresponding dilauroyl, dimyristoyl and dipalmitoyl compounds. A preferred liposome composition of the invention contains from 10 to 90 % by weight of helper. lipid, such as phospholipid, in combination with 10 to 90 % by weight of a sphingosine derivative. A more preferred composition is from 30 to 70 % by weight of helper lipid and 70 to 30 % by weight of a sphingosine derivative. As will be shown later, very good results have been obtained using DOPE and sphingosine in an amount of 1:1 (wt:wt), which corresponds to a molar ratio between the compounds of appr. 1:2.

According to a preferred embodiment of the invention, the cationic liposomal composition includes a glyceride, preferably a diacyl glycerol as a second helper lipid. According to the invention it has namely been shown that the transfection efficiency in terms of expression can be increased by incorporating an amount of diacyl glycerol in the composition. For the purpose of the invention, especially a diacyl glycerol selected from dioleoylglycerol and dioctanoylglycerol, are contemplated. Diacyl glycerol, when included as a second helper lipid, is included in an amount of up to 25 mole % calculated from the lipid composition of the liposome. Optimal results have been obtained using appr. 5 to 10 mole % of such diacyl glycerols (calculated of total lipids).

The preparation of the liposomes to be used according to the invention is well known to a person skilled in the art, see for example Kinnunen P. K. J., et al., *Chem. Phys. Lipids* 66 (1993) 75-85, describing the preparation of multilamellar liposomes. Briefly, sphingosine and any further lipids to be used are mixed in a suitable solvent, e.g. in chloroform, and evaporated to dryness under a stream of nitrogen. To prepare the liposomes, the thus dried lipid mixture is hydrated in a buffer, including short periodical vortexing, and sonicated at the end of the hydration period, to form multilamellar liposomes.

Nucleic acid includes in this context DNA and RNA, and oligonucleotides of DNA and RNA.

To prepare the nucleic acid-lipid complex, the nucleic acid and the lipids are each diluted in an appropriate medium, for example in serum-free DMEM (Dulbecco's Modified Eagle's Medium), mixed and preincubated for a suitable period of time. The amount of nucleic acid to lipid can vary, but a weight ratio between nucleic acid and lipid in the range of 0.25:15 to 4:5 has been found to be satisfactory. Due to charge interactions, the nucleic acid forms a strong complex with the cationic liposome surface after simple mixing of the components. Good transfection results have been obtained in tests using 1 or 2 µg respectively of DNA to 10 µg of total lipid. The concentration of nucleic acid in the transfection medium is suitably adjusted to 1 to 2 µg/ml.

The term "cell" means in this context an animal cell or a plant cell. Unicellular organisms as well as multicellular organisms or systems such as cell cultures are contemplated in the invention

Methods of transfection are well known in the art and such known methods can be used with the present liposome compositions as well. The invention thus concerns an improved method of transfection, the improvement comprising using, as the transfection vector, a liposome comprising a nucleic acid and a sphingosine derivative, and preferably a helper phospholipid.

The invention will be described in more detail in the following examples, referring to enclosed drawing, wherein

Figure 1 shows the effect of different liposomal combinations on the transfection efficiency of pCMV-luci in KK-1 cells. KK-1 cells were transfected with 10 µg lipid and 1 µg CMV-luci plasmid. After harvest, the luciferase activity of the cell lysates were measured. Buff= cells not transfected; 0 = cells transfected with 1 µg DNA only, with no liposomes; sph-PC = sphingosylphosphorylcholine; sph = sphingosine; phsph = phytosphingasine; DE = DOPE; DG = DOG; DOPE and sphingosine were mixed 1:1 (wt:wt) and 10 mole % of DOG was added were indicated.

Figure 2 shows the transfection efficiency of DOPE-phytosphingosine liposomes added with 5 mole % of either dioctanoylglycerol (DOcG) or dioleoylglycerol (DOG) and tested on HeLa and KK-1 cells. 10 µg of lipid and 2 µg (HeLa cells) or 1 µg (KK-1 cells) of DNA was used in the transfection. The expression was compared to that in the cells transfected with Lipofectin^{•}.

Figure 3a shows the effect of adding different molar percentages of DOG into DOPE-phytosphingosine liposomes. 10 µg of total lipid and 1 µg of DNA was used to transfect KK-1 cells.

Figure 3b shows the effect of combining different molar percentages of DOcG into DOPE-phytosphingosine liposomes. 10 µg of total lipid and 1 µg of DNA was used to transfect KK-1 cells.

Figure 4 shows the transfection efficiency using various DNA/total lipid ratios on KK-1 cells. The liposome composition was DOPE:phytosphingosine in a ratio of 1:1 (wt:wt) and 5 mol% of DOG.

Figure 5 shows the results of storage tests of liposomes carried out on KK-1 cells. The dark column represents storage for a composition according to the invention (composition as in Figure 4 except that DOcG was used instead of DOG) stored at +4 °C and the grey column that of the same composition stored at -20 °C. The striped column represents storage results for Lipofectin ^{•} stored at +4 °C according to the instructions of the manufacturer.

### EXAMPLES

### Preparation of a transfection liposome

Liposomes were prepared using lipid stocks of DOPE and of appropriate sphingosine derivative selected from sphingosine, phytosphingosine and phosphorylcholine-sphingosine. The lipids chosen were dissolved in chloroform at a ratio of 1:1 (by weight). To the solution obtained either dioleoylglycerol (DOG) or dioctanoylglycerol (DOcG) were added in indicated amounts, up to 30 mole %. The solvent was evaporated under a stream of nitrogen and the drying was continued in a lyophilizator for at least 2 hours or overnight. Thereafter the dry lipid mixture was hydrated in a buffer containing 20 mM Hepes, 150 mM NaCl, 0.1 EDTA, pH 7.4, for 30 minutes at 40°C in a water bath, and vortexed briefly every 5 minutes. At the end of hydration the liposomes obtained were sonicated for appr. 5 minutes. The total lipid concentration of the liposomes was usually 100 µg/ml buffer. The liposomes prepared were kept in a refrigerator, in closed test tubes.

Lipofectin^{•} was used as the reference liposome vector according to the instructions of the manufacturer.

### Preparation of DNA-lipid complexes

The pCMV-luciferase construct used in the experiments was designed to carry the promoter area of the cytomegalo virus in front of the firefly luciferase coding sequence in an expression vector PUHC13-1 (Gossen M., and Bujard H., *Proc. Natl. Acad. Sci.* 89 (1992), 5547-5551). The DNA preparation was purified twice with CsCl gradient centrifugation and dissolved in sterile water for the transfections. The identity of the CMV-luciferase construct was verified using the specific restriction endonuclease digestion.

DNA and the lipid were each diluted to 100 µl of serum free DMEM (DMEM-SF) and then mixed. 1 µg or 2 µg, respectively, of DNA and 10 µg of total lipid were pre-incubated for 15 minutes. Thereafter the total volume of the mixture was adjusted to 1 ml with DMEM-SF. The DNA-lipid complexes formed rapidly after mixing and had a stable transfectability at least for one hour at the concentration used.

### Transfection

As cell cultures the cell line KK-1 and HeLa cells were used.The KK-1 cells were derived from transgenic murine ovarian tumour cells immortalized by expression of the simian virus 40 large and small tumour (T) antigens under the control of inhibin-α subunit promoter (Kananen K., et al. *Mol. Endacrinol.* 9 (1995) 616-627). The HeLa cells are derived from human uterine cervix carcinoma (see e.g. Gossen, M. et al., *Proc. Natl. Acad. Sci. USA* 89 (1992) 5547-5551). The cells were grown on plastic tissue culture plates in Dulbecco's modified Eagle's medium with 10 % fetal bovine serum (DMEM-10) in an incubator in an atmosphere of 5 % CO₂ in air at 37 ° C.

At least 80 % confluent six-well cell culture plates were washed with DMEM-SF and the DNA-lipid complexes were laid over the cells. For each well 1 µg DNA/10 µg liposome was used for KK-1 cells and 2 µg/10 µg liposomes for HeLa cells. For each test, 2-3 parallel wells were used. After a 10 hour incubation period at 37°C, the DNA-lipid mixture was replaced by 2 ml of DMEM-10. Three days after beginning of transfection, the cells were harvested by scraping off the culture plates. The cell extracts were prepared and a luminometric assay for luciferase activity was performed by measuring the activity of the protein (the ATP luciferase enzyme) produced by the cells, by measuring the degradation of ATP by the luciferase enzyme (see e.g. Gould S., et al., *Anal. Biochem.* 7 (1988), 5-13; Nguyen V., *Anal. Biochem.* 171 (1988) 404-408. In brief, 50 µl of the total of 100 µl of the cell extracts were mixed with 360 µl of assay buffer by short vortexing in a disposable cuvette. The cuvettes were thereafter placed in a Bio-Orbit Luminometer 1252 (Bio-Orbit Ltd., Turku, Finland) and the luminesence was measured after adding 200 µl of luciferin solution. When the luciferase activity of a sample was exceeding the measuring range, a smaller amount (10 or 20 µl) of the cell extract was used at re-measurement.

In order to measure PKC stimulation, phorbol myristic acid (PMA) (1 % of moles of lipids) was added to the liposome solutions. Transfection and expression analysis were performed as described above.

### Results

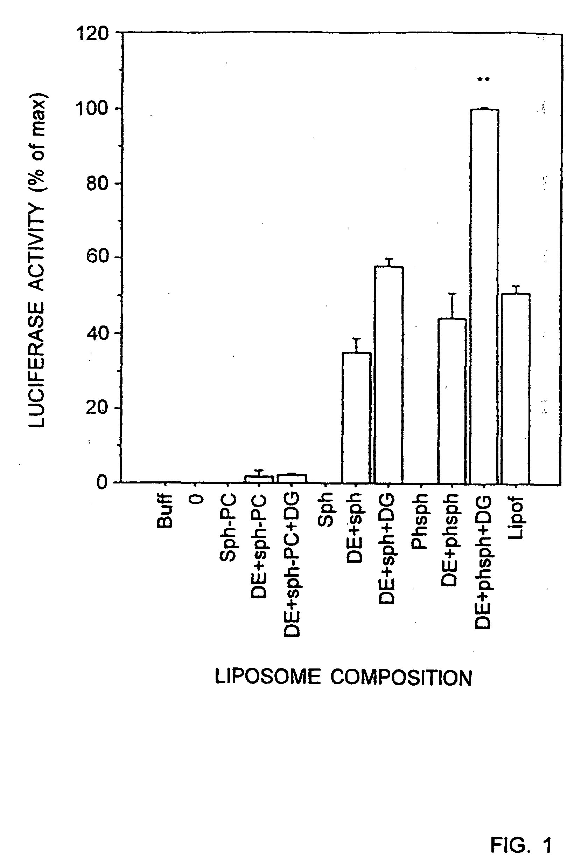

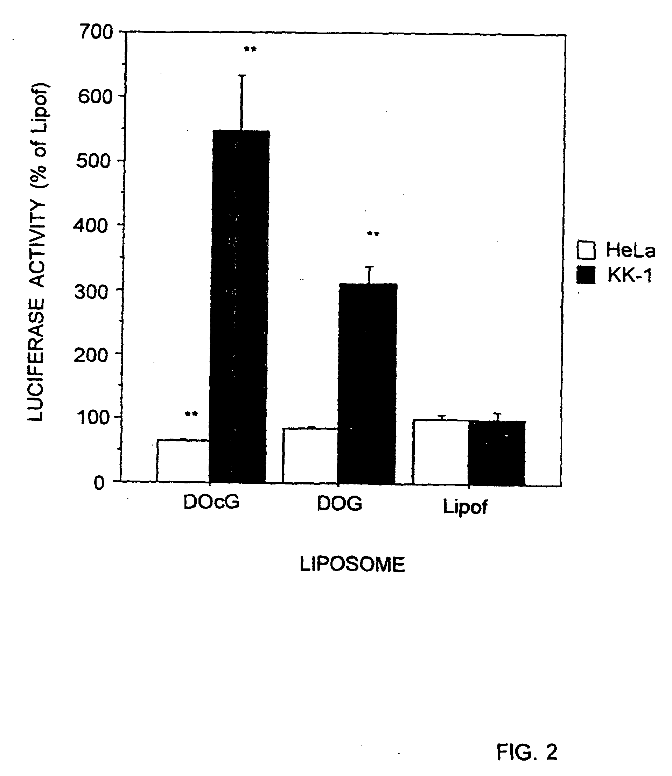

As is evident from the Fig. 1, the phytosphingosine and sphingosine containing liposomes showed major transfection efficiency in KK-1 cells comparable to or exceeding that of Lipofectin^{•}. The transfection efficiency is poor in compositions not containing DOPE. The transfection efficiency in HeLa cells was somewhat poorer than that in KK-1 cells, see Fig. 2.

After incubation of KK-1 cells with liposomes for 16 h or longer, the cells transfected with Lipofectin^{•} had almost all died, whereas the sphingosine liposomes showed no marked cell death. The HeLa cells tolerated better Lipofectin^{•} showing less damage after incubation periods over 10 hours.

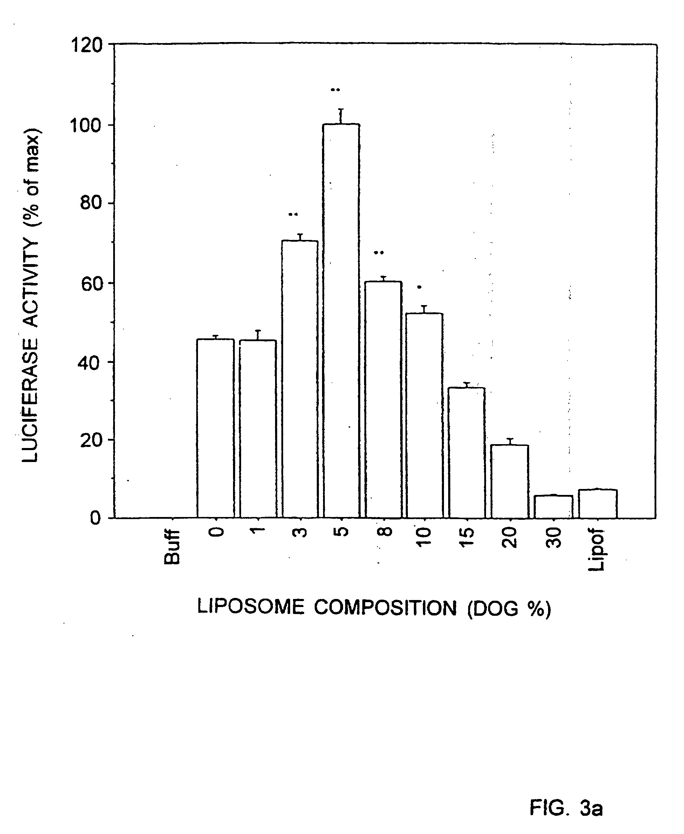

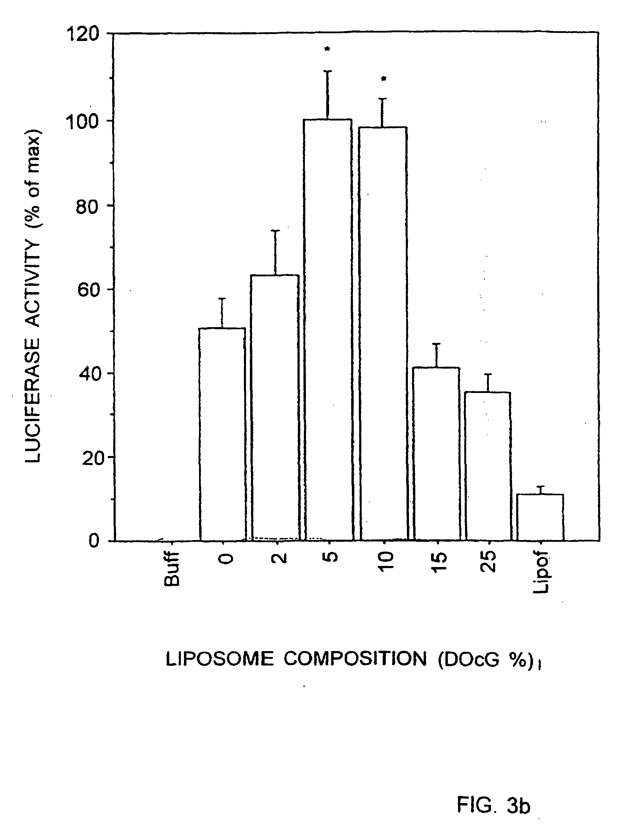

Adding diacylglycerol to the shingosine lipid composition affected the transient luciferase expression in a biphasical dose dependant manner (Fig. 3). From the results it is evident that dioleoylglycerol doubled the transfection at the optimal 5 % molar concentration, and that dioctanoylglycerol was most efficient at 5 to 10 % molar concentrations. Concentrations higher than appr. 25 molar % gave no improvement but rather caused lower expression. In wells stimulated by PMA, the expression was only a fourth of those not stimulated.

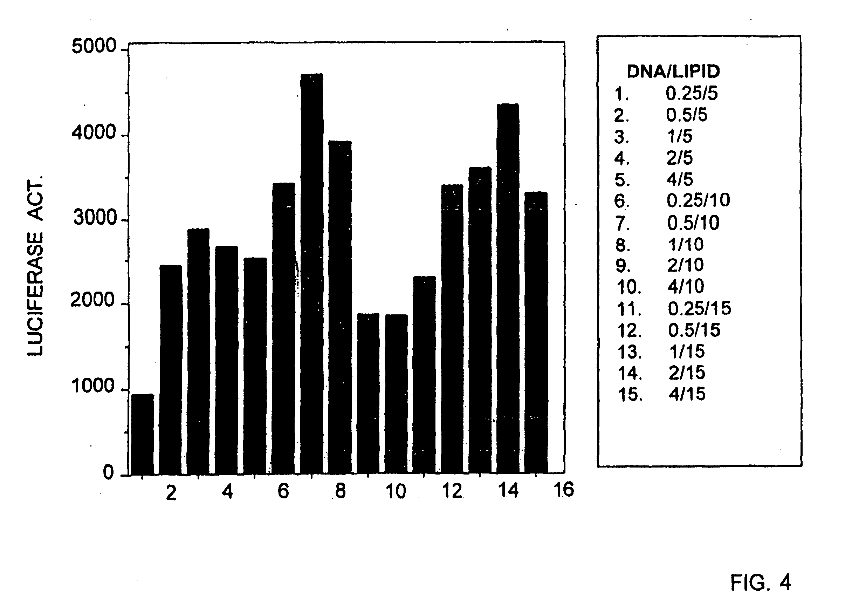

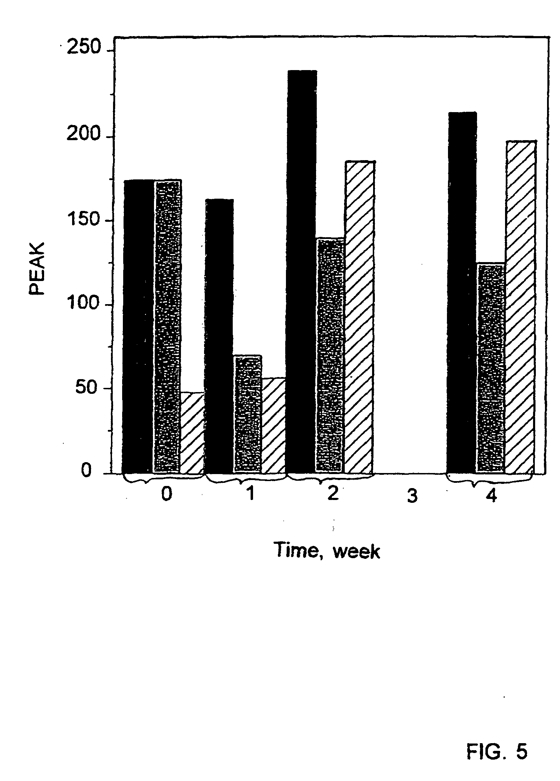

The results shown in Fig. 4 show that the DNA/lipid ratio is not very critical and that ranges from 0.25µg/15µg to 4µg/5µg gave satisfactory results. The storage test results of Fig. 5 indicate that the compositions for use according to the invention have at least comparable storage characteristics as compared to Lipofectin^{•}.

The results show that the toxicity of the sphingosine derivative containing liposomes on cells, such as KK-1 cells, apparently is lower than that of the widely used transfection liposomal preparation Lipofectin^{•}. It is contemplated that the non-biodegradabale DOTMA included in Lipofectin^{•} may account for the difference. Thus especially sensitive cell lines could more easily be handled with less toxic vectors. Some special cellular functions may also be disturbed in dying cells, e.g. promoter function experiments might be aberrated due to toxic effects. The PKC activators can increase the transfection efficiency by the calcium phosphate method (Reston J., et al., *Biochem. Biophys. Acta* 1088 (1991), 270-276). According to the invention, however, adding PMA to the liposome solution lowered the expression of the luciferase gene. The PKC activity thus seems to affect the sphingolipid liposome mediated transfection in opposite fashion, as compared with calcium phosphate transfection.

The availability and low cost of the components used according to the invention is a well acknowledged benefit in practice. The liposomes are easily prepared and the time consumption is modest. Hence the sphingosine derivative based liposomes are an efficient, low toxicity alternative for DNA transfection to cells in vitro. They may be of use also in transfection of other materials and in *in vivo* transfection.

## Claims

1. A method for in vitro-transfecting a cell with a nucleic acid, comprising contacting the cell with a transfection composition comprising the nucleic acid and liposomes, the nucleic acid being complexed to the surface of said liposomes, the liposomes comprising
- sphingosine or a derivative thereof, having a protonated amino group in the sphingosine moiety, and of the formula 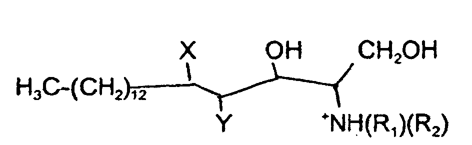 wherein
X and Y are each, independently of each other, H or OH, or together form a double bond, R₁ and R₂ are independently H or a lower alkyl group with 1-3 carbon atoms, and
- a helper lipid.

2. The method according to the claim 1, wherein the helper lipid is a neutral phospholipid.

3. The method according to the claim 2, wherein the phospholipid is a phosphatidylethanolamine.

4. The method according to the claim 2, wherein the composition comprises a diacyl glycerol as a further helper lipid:

5. The method according to the claim 1, wherein the composition comprises sphingosine, phytosphingosine, dihydrosphingosine or dimethylsphingosine.

6. The method according to the claim 2, wherein the phospholipid is dioleoylphosphatidylethanolamine (DOPE).

7. The method according to the claim 2, wherein the phospholipid is a phosphatidylethanolamine and the composition comprises a further helper lipid selected from the group consisting of dioleoyl- or dioctanoylglycerol.

8. The method according to the claim 2, wherein the composition further comprises a compound selected from cholesterol and sterol, and their derivatives.

9. The method according to the claim 1, wherein the composition comprises dioleoylphosphatidylethanolamine and a diacylglycerol selected from - dioleoylglycerol and dioctanoylglycerol.

10. The method according to the claim 1 or 2, wherein the composition contains sphingosine or a derivative thereof in an amount of 10 to 90 % by weight and a helper lipid in an amount from 10 to 90 % by weight, calculated from the combined weight of sphingosine or its derivative, and helper lipid.

11. The method according to the claim 1 or 2, wherein the composition contains sphingosine or a derivative thereof in an amount of 30 to 70 % by weight and helper lipid in an amount of from 30 to 70 % by weight, calculated from the combined weight of sphingosine or its derivative, and helper lipid.

12. The method according to the claim 1 or 2, wherein the ratio between sphingosine or a derivative thereof and the helper lipid is appr. 1:1 (wt/wt).

13. The method according to the claim 2, wherein the composition contains sphingosine or a derivative thereof in an amount of 30 to 70 % by weight and phospholipid in an amount of from 30 to 70 % by weight, calculated from the combined weight of sphingosine or its derivative and phospholipid, and a diacyl glycerol as a further helper lipid in an amount of up to 25 mole-% calculated on the total amount of lipids.

14. The method according to the claim 2, wherein the composition contains sphingosine or a derivative thereof in an amount of 30 to 70 % by weight and phospholipid in an amount of from 30 to 70 % by weight, calculated from the combined weight of sphingosine or its derivative, and phospholipid, and a diacyl glycerol as a further helper lipid in an amount of 5 to 10 mole-% of a diacylglycerol, calculated on the total amount of lipids.

15. The method according to the claim 1, wherein the ratio between nucleic acid and total lipids is 0.25:15 to 4:5 (wt/wt).

16. The method according to the claim 1, wherein the lipid composition comprises
- 30 to 70 % by weight of a compound selected from sphingosine and phytosphingosine
- 30 to 70 % by weight of dioleoylphosphatidylethanolamine, the said % by weight being calculated on the combined weight of sphingosine and phosphatidylethanolamine compounds, and in addition
- 5 to 15 mole %, calculated from the amount of total lipids, of a compound selected from dioleoylglycerol and dioctanoylglycerol.

17. Method for the preparation of a liposomal transfection composition for transfecting a cell with a nucleic acid, comprising complexing the nucleic acid to the surface of the liposomes, the liposomes comprising sphingosine or a derivative thereof, having a protonated amino group in the sphingosine moiety, and of the formula 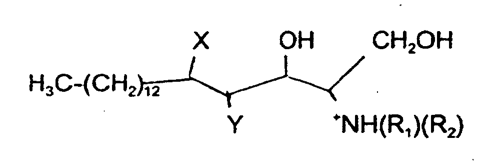 wherein
X and Y are each, independently of each other, H or OH, or together form a double bond, R₁ and R₂ are independently H or a lower alkyl group with 1-3 carbon atoms, and
- a helper lipid.

## Patentansprüche

1. Verfahren zur in vitro-Transfektion einer Zelle mit einer Nucleinsäure, umfassend Inkontaktbringen der Zelle mit einer Transfektionszusammensetzung, umfassend die Nucleinsäure und Liposomen, wobei die Nucleinsäure an die Oberfläche der Liposome komplexiert ist, wobei die Liposome umfassen
- Sphingosin oder ein Derivat davon, mit einer protonierten Aminogruppe in der Sphingosineinheit, und mit der folgenden Formel 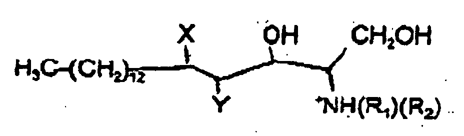 wobei
X und Y jeweils unabhängig voneinander eine H- oder OH-Gruppe darstellen oder zusammen eine Doppelbindung bilden, R₁ und R₂ unabhängig voneinander eine H-oder eine Niederalkylgruppe mit 1-3 Kohlenstoffatomen darstellen, und
- ein Helferlipid.

2. Verfahren nach Anspruch 1, wobei das Helferlipid ein neutrales Phospholipid ist.

3. Verfahren nach Anspruch 2, wobei das Phospholipid ein Phosphatidylethanolamin ist.

4. Verfahren nach Anspruch 2, wobei die Zusammensetzung ein Diacylglycerin als ein weiteres Helferlipid umfasst.

5. Verfahren nach Anspruch 1, wobei die Zusammensetzung Sphingosin, Phytosphingosin, Dihydrosphingosin oder Dimethylsphingosin umfasst.

6. Verfahren nach Anspruch 2, wobei das Phospholipid Dioleoylphosphatidylethanolamin (DOPE) ist.

7. Verfahren nach Anspruch 2, wobei das Phospholipid ein Phosphatidylethanolamin ist und die Zusammensetzung außerdem ein weiteres Helferlipid, ausgewählt aus der Gruppe bestehend aus Dioleoyl- oder Dioctanoylglycerin, umfasst.

8. Verfahren nach Anspruch 2, wobei die Zusammensetzung außerdem eine Verbindung umfasst, die ausgewählt ist aus Cholesterin und Sterol und ihren Derivaten.

9. Verfahren nach Anspruch 1, wobei die Zusammensetzung Dioleoylphosphatidylethanolamin und ein Diacylglycerin ausgewählt aus Dioleoylglycerin und Dioctanoylglyocrin enthält.

10. Verfahren nach Anspruch 1 oder 2, wobei die Zusammensetzung Sphingosin oder ein Derivat davon in einer Menge von 10 bis 90 Gew.-% und ein Helferlipid in einer Menge von 10 bis 90 Gew.-%, berechnet ausgehend vom gemeinsamen Gewicht von Sphingosin oder seinem Derivat und dem Helferlipid, enthält.

11. Verfahren nach Anspruch 1 oder 2, wobei die Zusammensetzung Sphingosin oder ein Derivat davon in einer Menge von 30 bis 70 Gew.-% und eln Helterlipid in einer Menge von 30 bis 70 Gew.-%, berechnet ausgehend vom gemeinsamen Gewicht von Sphingosin oder seinem Derivat und dem Helferlipid, enthält.

12. Verfahren nach Anspruch 1 oder 2, wobei das Verhältnis zwischen Sphingosin oder einem Derivat davon und dem Helferlipid ungefähr 1:1 (wt/wt) ist.

13. Verfahren nach Anspruch 2, wobei die Zusammensetzung Sphingosin oder ein Derivat davon in einer Menge von 30 bis 70 Gew.-% und Phospholipid in einer Menge von 30 bis 70 Gew.-%, berechnet ausgehend vom gemeinsamen Gewicht von Sphingosin oder seinem Derivat und Phospholipid, und ein Diacylglycerin als weiteres Helferlipid in einer Menge von bis zu 25 Mol-%, gerechnet ausgehend vom Lipidgesamtgewicht, enthält.

14. Verfahren nach Anspruch 2, wobei die Zusammensetzung Sphingosin oder ein Derivat davon in einer Menge von 30 bis 70 Gew.-% und Phospholipid in einer Menge von 30 bis 70 Gew.-%, berechnet ausgehend vom gemeinsamen Gewicht von Sphingosin oder seinem Derivat und Phospholipid, und ein Diacylglycerin als weiteres Helferlipid in einer Menge von 5 bis 10 Mol-% eines Diacylglycerin, berechnet ausgehend vom Lipidgesamtgewicht, enthält.

15. Verfahren nach Anspruch 1, wobei das Verhältnis zwischen der Nucleinsäure und der Gesamtheit der Lipide 0,25:15 bis 4:5 (Gew./Gew.) beträgt.

16. Verfahren nach Anspruch 1, wobei die Lipidzusammensetzung enthält
- 30 bis 70 Gew.% einer Verbindung ausgewählt aus Sphingosin und Phytosphingosin
- 30 bis 70 Gew.-% Dioleoylphosphatidylethanolamin, wobei das Gew.-% berechnet wird ausgehend vom Gesamtgewicht von Sphingosin und Phosphatidylethanolamin-Verbindungen, und zusätzlich
- 5 bis 15 Mol-%, berechnet ausgehend von der Lipidgesamtmenge, von einer Verbindung ausgewählt aus Dioleoylglycerin und Dioctanoylglycerin.

17. Verfahren für die Herstellung einer liposomalen Transfektionszusammensetzung zur Transfektion einer Zelle mit einer Nucleinsäure, umfassend die Komplexierung der Nucleinsäure an die Oberfläche der Liposomen, wobei die Liposomen Sphingosin oder ein Derivat davon umfassen, mit einer protonierten Aminogruppe in der Sphingosineinheit und mit der Formel 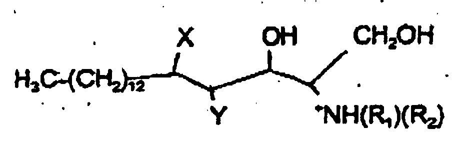 wobei X und Y jeweils unabhängig voneinander eine H- oder OH-Gruppe darstellen oder gemeinsam eine Doppelbindung bilden, R₁ und R₂ unabhängig eine H- oder eine Niederalkylgruppe mit 1-3 Kohlenstoffatomen darstellen, und
- ein Helferlipid.

## Revendications

1. Procédé de transfection in vitro d'une cellule avec un acide nucléique, comprenant de mettre en contact la cellule avec une composition de transfection comprenant l'acide nucléique et des liposomes, l'acide nucléique formant un complexe avec la surface desdits liposomes, les liposomes comprenant :
de la sphingosine ou un dérivé de celle-ci, ayant un groupe amino protoné dans le fragment de sphingosine, et de formule : 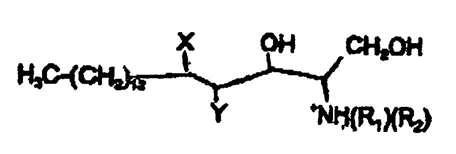 dans laquelle
X et Y sont chacun, indépendamment l'un de l'autre, H ou OH, ou ensemble forment une double liaison, R₁ et R₂ sont indépendamment H ou un groupe alkyle inférieur avec 1 à 3 atomes de carbone, et
un lipide accessoire.

2. Procédé selon la revendication 1, dans lequel le lipide accessoire est un phospholipide neutre.

3. Procédé selon la revendication 2, dans lequel le phospholipide est une phosphatidyl-éthanolamine.

4. Procédé selon la revendication 2, dans lequel la composition comprend un diacyl glycérol comme autre lipide accessoire.

5. Procédé selon la revendication 1, dans lequel la composition comprend de la sphingosine, de la phytosphingosine, de la dihydrosphingosine ou de la diméthylsphingosine.

6. Procédé selon la revendication 2, dans lequel le phospholipide est la dioléoyl phosphatidyl-éthanolamine (DOPE).

7. Procédé selon la revendication 2, dans lequel le phospholipide est une phosphatidyl-éthanolamine et la composition comprend un autre lipide accessoire choisi dans le groupe composé de dioléoyl glycérol ou dioctanoyl glycérol.

8. Procédé selon la revendication 2, dans lequel la composition comprend en outre un composé choisi parmi le cholestérol et le stérol, et leurs dérivés.

9. Procédé selon la revendication 1, dans lequel la composition comprend de la dioléoyl phosphatidyl-éthanolamine et un diacyl glycérol choisi parmi le dioléoyl glycérol et le diocténoyl glycérol.

10. Procédé selon la revendication 1 ou 2, dans lequel la composition contient de la sphingosine ou un dérivé de celle-ci en quantité comprise entre 10 et 90 % en poids et un lipide accessoire en quantité comprise entre 10 et 90 % en poids, calculées à partir des poids combinés de la sphingosine ou son dérivé et du lipide accessoire.

11. Procédé selon la revendication 1 ou 2, dans lequel la composition contient de la sphingosine ou un dérivé de celle-ci en quantité comprise entre 30 et 70 % en poids et un lipide accessoire en quantité comprise entre 30 et 70 % en poids, calculées à partir des poids combinés de la sphingosine ou son dérivé et du lipide accessoire.

12. Procédé selon la revendication 1 ou 2, dans lequel le rapport entre la sphingosine ou un dérivé de celle-ci et le lipide accessoire est d'environ 1/1 (poids/poids).

13. Procédé selon la revendication 2, dans lequel la composition contient de la sphingosine ou un dérivé de celle-ci en quantité comprise entre 30 et 70 % en poids et un phospholipide en quantité comprise entre 30 et 70 % en poids, calculées à partir des poids combinés de la sphingosine ou son dérivé et du phospholipide, et un diacyl glycérol comme autre lipide accessoire en quantité allant jusqu'à 25 mol % calculée sur la quantité totale de lipides.

14. Procédé selon la revendication 2, dans lequel la composition contient de la sphingosine ou un dérivé de celle-ci en quantité comprise entre 30 et 70 % en poids et un phospholipide en quantité comprise entre 30 et 70 % en poids, calculées à partir des poids combinés de la sphingosine ou son dérivé et du phospholipide, et un diacyl glycérol comme autre lipide accessoire en quantité comprise entre 5 et 10 mol % d'un diacyl glycérol, calculée sur la quantité totale de lipides.

15. Procédé selon la revendication 1, dans lequel le rapport entre l'acide nucléique et la totalité des lipides est compris entre 0,25/15 et 4/5 (poids/poids).

16. Procédé selon la revendication 1, dans lequel la composition de lipides comprend :
30 à 70 % en poids d'un composé choisi parmi la sphingosine et la phytosphingosine,
30 à 70 % en poids de dioléoyl phosphatidyl-éthanolamine, ledit pourcentage en poids étant calculé sur les poids combinés des composés sphingosine et phosphatidyl-éthanolamine, et en plus :
5 à 15 mol %, calculés à partir de la quantité de la totalité des lipides, d'un composé choisi parmi le dioléoyl glycérol et le dioctanoyl glycérol.

17. Procédé pour la préparation d'une composition de transfection liposomale pour transfecter une cellule avec un acide nucléique, comprenant de complexer l'acide nucléique à la surface des liposomes, les liposomes comprenant de la sphingosine ou un dérivé de celle-ci, ayant un groupe amino protoné dans le fragment de sphingosine, et de formule 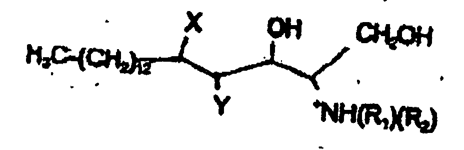 dans laquelle
X et Y sont chacun, indépendamment l'un de l'autre, H ou OH, ou ensemble forment une double liaison, R₁ et R₂ sont indépendamment H ou un groupe alkyle inférieur avec 1 à 3 atomes de carbone, et un lipide accessoire.
